# EUROPEAN PATENT SPECIFICATION

(11) **EP 3 778 874 B1**
(45) Date of publication and mention of the grant of the patent: **28.01.2026**
(21) Application number: 19782003.8
(22) Date of filing: 02.04.2019
(51) Int. Cl.: C12N 5/074

(54) **COMPOSITION FOR INDUCING DEDIFFERENTIATION FROM SOMATIC CELLS TO INDUCED PLURIPOTENT STEM CELLS AND METHOD OF INDUCING DEDIFFERENTIATION USING SAME**
ZUSAMMENSETZUNG ZUR INDUKTION DER DEDIFFERENZIERUNG VON SOMATISCHEN ZELLEN IN INDUZIERTE PLURIPOTENTE STAMMZELLEN UND VERFAHREN ZUR INDUKTION DER DEDIFFERENZIERUNG UNTER VERWENDUNG DERSELBEN
COMPOSITION POUR INDUIRE UNE DÉDIFFÉRENCIATION À PARTIR DE CELLULES SOMATIQUES EN CELLULES SOUCHES PLURIPOTENTES INDUITES ET MÉTHODE D'INDUCTION DE DÉDIFFÉRENCIATION L'UTILISANT

(30) Priority: 02.04.2018 KR 20180038371; 23.07.2018 KR 20180085476
(43) Date of publication of application: 17.02.2021
(73) Proprietor: Korea University Research and Business Foundation, Seoul 02841 (KR)
(72) Inventor: KIM, Byung Soo, Seoul 06005 (KR); LEE, Seung Jin, Seoul 03414 (KR)
(74) Representative: J A Kemp LLP
(86) International application number: PCT/KR2019/003898
(87) International publication number: WO 2019/194549

(56) References cited:
- WO-A1-2016/076507
- WO-A1-92/18641
- KR-A- 20110 078 234
- KR-A- 20170 019 696
- KR-B1- 101 395 214
- US-A1- 2009 047 263
- US-B1- 9 580 682
- JI-HYE JUNG ET AL: "CXCR2 and Its Related Ligands Play a Novel Role in Supporting the Pluripotency and Proliferation of Human Pluripotent Stem Cells", STEM CELLS AND DEVELOPMENT, vol. 24, no. 8, 15 April 2015 (2015-04-15), US, pages 948 - 961, XP055755854, ISSN: 1547-3287, DOI: 10.1089/scd.2014.0381
- JI-HYE JUNG ET AL: "CXCR2 Inhibition in Human Pluripotent Stem Cells Induces Predominant Differentiation to Mesoderm and Endoderm Through Repression of mTOR, [beta]-Catenin, and hTERT Activities", STEM CELLS AND DEVELOPMENT, vol. 25, no. 13, 1 July 2016 (2016-07-01), US, pages 1006 - 1019, XP055755853, ISSN: 1547-3287, DOI: 10.1089/scd.2015.0395
- TAOTAO CHEN ET AL: "Rapamycin and other longevity-promoting compounds enhance the generation of mouse induced pluripotent stem cells", AGING CELL, vol. 10, no. 5, 14 June 2011 (2011-06-14), pages 908 - 911, XP055078410, ISSN: 1474-9718, DOI: 10.1111/j.1474-9726.2011.00722.x
- PARK YONG ET AL: "Human Feeder Cells Can Support the Undifferentiated Growth of Human and Mouse Embryonic Stem Cells Using Their Own Basic Fibroblast Growth Factors", STEM CELLS AND DEVELOPMENT, vol. 20, no. 11, 1 November 2011 (2011-11-01), US, pages 1901 - 1910, XP055800050, ISSN: 1547-3287, DOI: 10.1089/scd.2010.0496
- LEE SEUNG-JIN ET AL: "CXCR2 Ligands and mTOR Activation Enhance Reprogramming of Human Somatic Cells to Pluripotent Stem Cells", STEM CELLS AND DEVELOPMENT, vol. 29, no. 3, 6 December 2019 (2019-12-06), US, pages 119 - 132, XP055799879, ISSN: 1547-3287, DOI: 10.1089/scd.2019.0188
- BYUNG SOO KIM: "Production of Conditioned Medium Derived front Standardization Human Cell for Complete Feeder Free Culture of Human iPSCs and Its Mechanism Study Ministry of Science and ICT", 13 November 2017 (2017-11-13), pages 1 - 32, XP009524177, Retrieved from the Internet <URL:https://scienceon.kisti.re.kr/srch/selectPORSrchReport.do?cn=TRKO201800002943>

## Description

### Technical Field

The present invention was made by project number 2017M3A9C6027001 under the support of the Ministry of Science and Technology of the Republic of Korea. The research management institution for the above project is the Korea Research Foundation, the name of the research project is "Bio. Medical Technology Development Project", and the name of the research project is "Direct Cross Differentiation Human Neural Stem Cell Culture Production Technology Optimization and Stability Study". The host institution is Korea University, and the research period is from April 01, 2017 to March 31, 2021.

The present disclosure relates to use of a composition for increasing the efficiency of dedifferentiation from a somatic cell to an induced pluripotent stem cell (iPS), wherein the composition comprises CXCR2(CXC chemokine receptor 2), which is a receptor on a human somatic cell. The invention also relates to an *in vitro* method for increasing the efficiency of dedifferentiation from a somatic cell to an induced pluripotent stem cell (iPS).

### Background Art

The production of a stem cell therapy product indispensably requires *in vitro* mass culture of a stem cell, which is a source therefor, with the safety and economical benefit of the cultured stem cell requisite for clinical application as the cell therapy product.

Nowadays, however, a human pluripotent stem cell needs an animal-derived feeder cell for the proliferation and culture thereof or employs a vessel coated with a special gel containing an animal-derived product for the growth thereof. Thus, there is an ever-present danger of contamination with heteroproteins, which leads to a concern with safety, and the employment of an expensive special gel is not economically adequate for mass production of the cell therapy product.

Attempts have been made to use versatile viruses, chemicals, cytokines, etc. in increasing the production efficiency of a dedifferentiated stem cell that could be clinically applied in future. For example, Sendai virus is used so as to prevent integration into host chromosomes or there are studies taking advantage of fusion protein transduction or mRNA.

In relation to conventional preparation methods for an induced pluripotent stem cell (iPS) by reprogramming, there is the problem of low efficiency that only about 10 iPSs are obtained from as many as 5x10⁴ human dermal fibroblast cells.

KR 101 395 214 B1 relates to placenta-derived cells conditioned media and animal-free, feeder-free culture method for maintaining undifferentiated stem cells using the same. Jung et al. (Stem cells and development 24.8 (2015): 948-961) relates to CXCR2 and its related ligands playing a novel role in supporting the pluripotency and proliferation of human pluripotent stem cells. Jung et al. (Stem cells and development 25.13 (2016): 1006-1019) relates to CXCR2 inhibition in human pluripotent stem cells inducing predominant differentiation to mesoderm and endoderm through repression of mTOR, [beta]-Catenin, and hTERT activities. Chen, et al. (Aging cell 10.5 (2011): 908-911) relates to Rapamycin and other longevity-promoting compounds enhancing the generation of mouse induced pluripotent stem cells.

### Summary of the invention

The invention provides use of a composition comprising CXCR2 (CXC chemokine receptor 2) or a ligand thereof for increasing the efficiency of dedifferentiation from a somatic cell to an induced pluripotent stem cell (iPS), wherein the ligand of CXCR2 is at least one selected from the group consisting of GRO-α (growth-regulated oncogene-α), GRO-β (growth-regulated oncogene-β), GRO-γ (growth-regulated oncogene-γ), GCP-2 (granulocyte chemotactic protein-2), NAP-2 (Neutrophil Activating Peptide-2), ENA-78 (Epithelial neutrophil-activating protein-78), and IL-8 (Interleukin-8).

The invention also provides an *in vitro* method for increasing the efficiency of dedifferentiation from a somatic cell to an induced pluripotent stem cell (iPS), the method comprising: a somatic cell transformation step of increasing the expression of CXCR2; and a somatic cell culturing step of culturing the transformed somatic cell.

### Detailed Description of the Invention

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

As a result of thorough and intensive research, the present inventors found that an excellent efficiency of dedifferentiation into an induced pluripotent stem cell (iPS) can be obtained when somatic cells transformed with CXCR2 (CXC chemokine receptor 2) ligand is dedifferentiated and when dedifferentiation is made in a somatic cell in which the expression level of CXCR2 has been increased by transformation.

The present disclosure relates to the use of a composition for increasing the efficiency of dedifferentiation from a somatic cell to an induced pluripotent stem cell (iPS). The composition and the *in vitro* method described herein increase an efficiency of dedifferentiation from a somatic cell to an induced pluripotent stem cell by stimulating CXCR2 (CXC chemokine receptor 2), which is a receptor on a human somatic cell.

Thorough and intensive research conducted by the present inventors results in the finding that dedifferentiation into an induced pluripotent stem cell (iPS) was conducted at increased efficiency when a somatic cell was cultured in a medium containing a CXCR2 ligand and when CXCR2 expression was upregulated through transformation, but at decreased efficiency when the expression of CXCR2 in the somatic cell was downregulated.

Below, a detailed description will be given of the present disclosure.

An aspect of the present disclosure pertains to a use of a composition comprising CXCR2 (CXC chemokine receptor 2) or a ligand thereof for increasing the efficiency of dedifferentiation from a somatic cell to an induced pluripotent stem cell (iPS).

CXC chemokine receptors are an integral membrane protein that specifically bind and respond to cytokines of the CXC chemokine family. Among them, CXCR2 is a closely related receptor that recognizes CXC chemokines that possess an E-L-R amino acid motif immediately adjacent to the CXC motif thereof. Chemokines are a family of cytokine proteins known to induce directed chemotaxis.

The ligand of CXCR2 is at least one selected from the group consisting of GRO-α (growth-regulated oncogene-α), GRO-β (growth-regulated oncogene-β), GRO-γ (growth-regulated oncogene-γ), GCP-2 (granulocyte chemotactic protein-2), NAP-2 (Neutrophil Activating Peptide-2), ENA-78 (Epithelial neutrophil-activating protein-78), and IL-8 (Interleukin-8).

The somatic cell may be at least one selected from the group consisting of endothelial cells, epithelial cells, and placenta cells.

The composition may further comprise a placenta-derived cell conditioned medium.

As used herein, the term "placenta-derived cell conditioned medium" refers to a medium prepared by inoculating placenta-derived cells into a gelatin-coated well plate, adding a cell culture to the plate to culture the placenta-derived cells, and collecting the supernatant only. When used, the human placenta-derived feeder cells were found to allow human embryonic stem cells to remain undifferentiated and thus have arisen for the utility thereof.

The placenta-derived cell may be a placenta-derived fibroblast-like cell that is isolated from human chorionic plate and cultured.

The placenta-derived cell conditioned medium may contain human placenta-derived cells cultured in a cell growth medium.

A further aspect of the present disclosure pertains to an *in vitro* method for increasing dedifferentiation from a somatic cell to an induced pluripotent stem cell (iPS), the method comprising:
a somatic cell transformation step of increasing expression of CXCR2; and
a somatic culture step of culturing the transformed somatic cell.
In the somatic cell transformation step, a CRISPR/Cas nuclease system or lentiviral activation particles may be used.

As used herein, the term "lentivirus" refers to a genus of retroviruses, a kind of RNA viruses which translocate into the nucleus of a host cell and integrate into the host DNA to express the viral cDNA and produce viral particles. Lentivirus is used as a vector for regulating gene expression by virtue of taking advantage of such mechanisms.

The somatic cell transformation step may be conducted by infecting CXCR2 lentiviral activation particles into a somatic cell to upregulate CXCR2 expression therein.

The somatic cell may be at least one selected from the group consisting of an endothelial cell, an epithelial cell, and a placenta cell.

The culture may be performed in a placenta-derived cell conditioned medium.

The placenta-derived cell may be a placenta-derived fibroblast-like cell that is isolated from human chorionic plate and cultured.

The placenta-derived cell conditioned medium may contain human placenta-derived cells cultured in a cell growth medium.

The dedifferentiation induction method may further comprise a stem cell isolation step of isolating a stem cell from the colony formed in the somatic cell culture step.

### Advantageous Effects

The present invention relates to a use of a composition for increasing the efficiency of dedifferentiation from a somatic cell to an induced pluripotent stem cell (iPS) and an *in vitro* method for increasing the efficiency of dedifferentiation from a somatic cell to an induced pluripotent stem cell (iPS). The dedifferentiation induction composition and method stimulate the human somatic cell receptor CXCR2 (CXC chemokine receptor 2) to increase efficiency of the dedifferentiation from somatic cells to induced pluripotent stem cells, finding effective applications in increasing the efficiency of dedifferentiation to induced pluripotent stem cells.

### Brief Description of the Drawings

FIG. 1a shows changes in expression levels of GRO-α (growth-regulated oncogene-α) in endothelial cells, epithelial cells, and placenta cells with culture time as measured by enzyme immunoassay.
FIG. 1b shows changes in expression levels of IL-8 (Interleukin-8) in endothelial cells, epithelial cells, and placenta cells with culture time as measured by enzyme immunoassay.
FIG. 1c shows comparison of GRO-α expression in cultured endothelial cells, epithelial cells, and placenta cells by immunofluorescence staining.
FIG. 1d shows comparison of IL-8 expression in cultured endothelial cells, epithelial cells, and placenta cells at mRNA levels.
FIG. 2a shows images of cells that have been induced to dedifferentiate from endothelial cells, epithelial cells, and placenta cells and then have undergone alkaline phosphatase staining to examine characteristics of induced pluripotent stem cells (iPS).
FIG. 2b is a graph showing characteristics of induced pluripotent stem cells that have dedifferentiated from endothelial cells, epithelial cells, and placenta cells and have undergone alkaline phosphatase staining.
FIG. 3a shows immunofluorescence images of cells in the stem cell dedifferentiation process from human endothelial cells to colony formation.
FIG. 3b shows immunofluorescence images of cells in the stem cell dedifferentiation process from human epithelial cells to colony formation.
FIG. 3c shows immunofluorescence images of cells in the stem cell dedifferentiation process from human placenta cells to colony formation.
FIG. 4a shows CXCR2 (CXC chemokine receptor 2) protein levels in cells dedifferentiated from endothelial cells in which CXCR2 expression has been downregulated using shRNA, as measured by western blotting.
FIG. 4b is a graph showing CXCR2 RNA expression levels in cells dedifferentiated from endothelial cells in which CXCR2 expression has been downregulated using shRNA.
FIG. 5a shows images of induced pluripotent stem cells after endothelial cells in which CXCR2 expression was downregulated using shRNA have been induced to differentiate and undergone alkaline phosphatase staining to examine dedifferentiation efficiency.
FIG. 5b is a graph showing efficiency of the dedifferentiation of induced pluripotent stem cells from endothelial cells in which CXCR2 expression has been downregulated using shRNA, as assayed by alkaline phosphatase staining.
FIG. 5c shows expression levels of CXCR2, mTOR, and β-Catenin, and expression levels of markers of induced pluripotent stem cells upon dedifferentiation of induced pluripotent stem cells from endothelial cells in which CXCR2 expression has been downregulated using shRNA, as measured by western blotting.
FIG. 6a shows CXCR2 protein levels in cells dedifferentiated from epithelial cells in which CXCR2 has been overexpressed using lentiviral activation particles, as measured by western blotting.
FIG. 6b is a graph of CXCR2 RNA expression levels in cells dedifferentiated from epithelial cells in which CXCR2 has been overexpressed using lentiviral activation particles.
FIG. 6c shows images of induced pluripotent stem cells obtained by inducing dedifferentiation from epithelial cells in which CXCR2 has been overexpressed using lentiviral activation particles.
FIG. 6d is a graph showing counts of colonies of induced pluripotent stem cells obtained by inducing dedifferentiation from epithelial cells in which CXCR2 has been overexpressed using lentiviral activation particles.

### EXAMPLES

### EXAMPLE 1: Assay for Expression of GRO-α and IL-8 in Endothelial Cells, Epithelial cells, and Placenta Cells

Primary umbilical endothelial cells (HUVEC) were purchased as the human endothelial cells from ATCC. An endothelial cell growth medium (LONZA), which contains 2% fetal bovine serum (FBS) and vascular endothelial growth factor (VEGF), was purchased. Cells were cultured in an incubator maintained at 37°C in a 5% CO₂ atmosphere.

Primary dermal fibroblasts (HDF) were purchased as the human epithelial cells from ATCC and cultured in a medium containing 90 % Dulbecco's modified Eagle's medium (DMEM) and 10 % FBS in a 5% CO₂ incubator maintained at 37°C.

Cells isolated from chorionic plate were used as the human placenta cells (HPC) and the isolated placenta cells were cultured in DMEM supplemented 10% FBS in a 5% CO₂ incubator maintained at 37°C.

In order to measure secretion levels of GRO-α and IL-8 therefrom, the endothelial cells, the epithelial cells, and the placenta cells were each cultured in culture dishes for 24, 48, and 72 hours and the culture media were collected and subjected to enzyme immunoassay to determine secretion levels of GRO-α and IL-8 with time.

In brief, the experiment was preformed using a kit (Human ELISA kit, Abcam). As for the experiment process, 100 µl of each of the samples was incubated overnight at 4°C and then incubated with a biotin antibody for one hours. After reaction with a streptavidin solution and a reagent (TMB One-Step Substrate Reagent), 50 µl of a stop solution was added to each of the sample to terminate the reaction. Absorbance was read at 450 nm on a microplate reader.

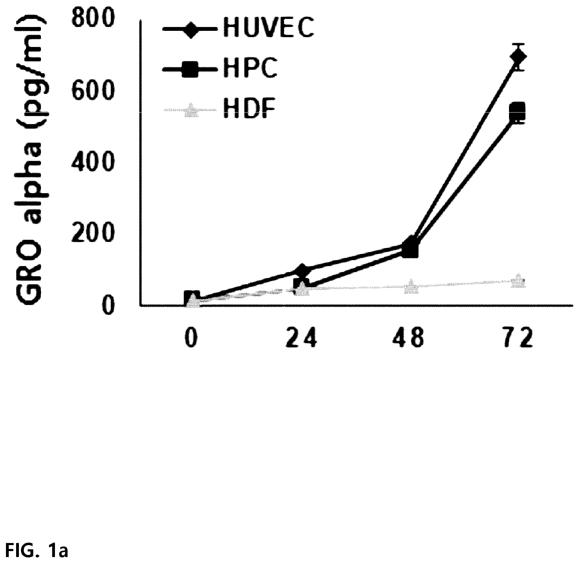

As can be seen in FIG. 1a and Table 1, GRO-α was expressed at higher levels in placenta cells and endothelial cells, especially in endothelial cells than in epithelial cells.

**TABLE 1**

| Time (hour) | Endothelial cell (HUVEC) | Placenta cell (HPC) | Epithelial cell (HDF) |
|---|---|---|---|
| 0 | 15.13 | 15.91 | 13.69 |
| 24 | 95.64 | 47.91 | 49.60 |
| 48 | 173.52 | 152.35 | 53.81 |
| 72 | 693.35 | 538.40 | 70.92 |

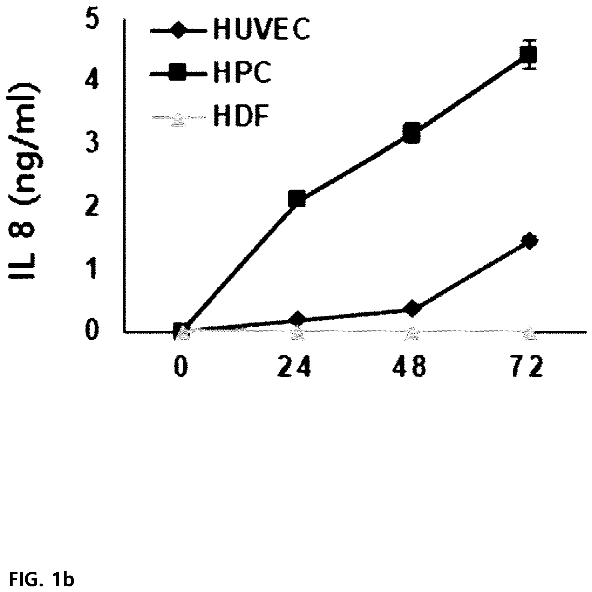

As can be seen in FIG. 1b and Table 2, higher expression levels of IL-8 were detected in placenta cells than in epithelial cells and endothelial cells.

**TABLE 2**

| Time (hour) | Endothelial cell (HUVEC) | Placenta cell (HPC) | Epithelial cell (HDF) |
|---|---|---|---|
| 0 | 0.00 | 0.00 | 0.00 |
| 24 | 0.19 | 2.11 | 0.00 |
| 48 | 0.36 | 3.17 | 0.00 |
| 72 | 1.45 | 4.42 | 0.00 |

The endothelial cell, epithelial cells, and placenta cells cultured in culture dishes were assayed for the expression of GRO-α by fluorescent staining.

Briefly, the cultured cells were fixed with 4% formalin at room temperature for 10 minutes, permeabilized with 0.1% Triton X-100/PBS for 10 minutes, and then incubated overnight with a primary antibody to GRO-α (Abcam #ab86436) at 4°C. On the next day, incubation with the secondary antibody Alexa Fluor^{®} 488 at room temperature for one hour was followed by treatment with 4',6-diamindino-2-phenylindone (DAPI) for 5 minutes in a dark condition before fluorescence microscopy to measure the expression of GRO-α in the cells.

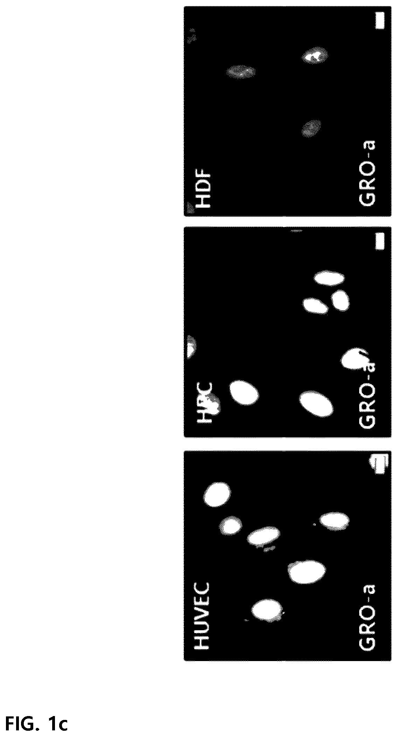

As shown in FIG. 1c, higher expression levels of GRO-α were detected in endothelial cells than in epithelial cells and placenta cells.

The expression of IL-8 gene in endothelial cells, epithelial cells, and placenta cells was compared and analyzed using real-time polymerase chain reaction. With the aid of a kit (Qiagen RNeasy kit, Qiagen Hilden, Germany), RNA was isolated from the differentiation-induced cells. In the presence of a reverse transcriptase (Superscript II reverse transcriptase, Gibco) and oligo(dT), 2 µg of RNA was used to synthesize cDNA.

For assay, the synthesized cDNAs were each polymerized using primers for IL-8 gene and a master mix (iQ SYBR Green qPCR Master Mix) in an apparatus (Bio-Rad iCycler iQ system, Bio-Rad Laboratories, USA). The assay values were normalized using GAPDH gene, and P values were used to determine statistical significance (**P =0.05*). Primers for IL-8 gene expression assay are as listed in Table 3, below.

**TABLE 3**

| SEQ ID NO: | Name | Sequence |
|---|---|---|
| 1 | IL-8 forward primer | CTGGCCGTGGCTCTCTTG |
| 2 | IL-8 reverse primer | CCTTGGCAAAACTGCACCTT |

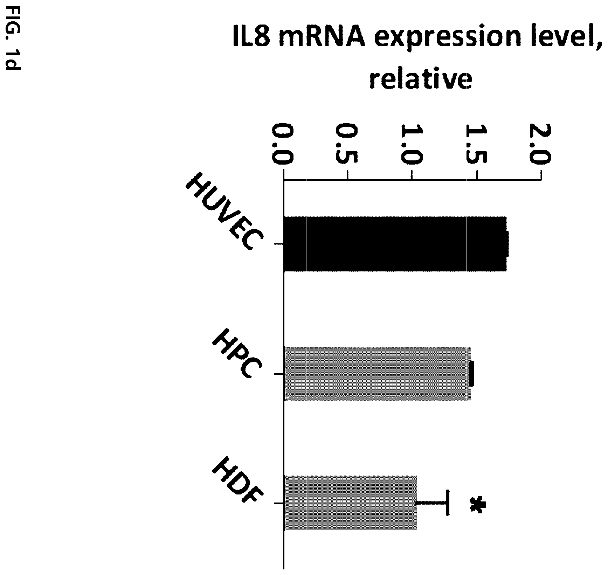

As is understood from the data of FIG 1d and Table 4, endothelial cells expressed GRO-α at higher levels than epithelial cells and placenta cells.

**TABLE 4**

| | Endothelial cell (HUVEC) | Placenta cell (HPC) | Epithelial cell (HDF) |
|---|---|---|---|
| Relative mRNA expression level of IL-8 | 1.72 | 1.45 | 1.03 |

As a consequence, GRO-α and IL-8 were expressed at the highest levels in endothelial cells and stimulated CXCR2, leading to dedifferentiation of endothelial cells at higher efficiencies than epithelial cells and placenta cells.

### EXAMPLE 2: Comparison of Dedifferentiation Efficiency among Endothelial Cell, Epithelial Cell, and Placenta Cell

Through alkaline phosphatase staining, an examination was made to see whether the stem cells dedifferentiated from the somatic cells in Example 1 possessed self-renewal, an ability typical characteristic for induced pluripotent stem cells. Alkaline phosphatase (ALP) staining was conducted using a kit (ES Cell Characterization kit, Chemicon International).

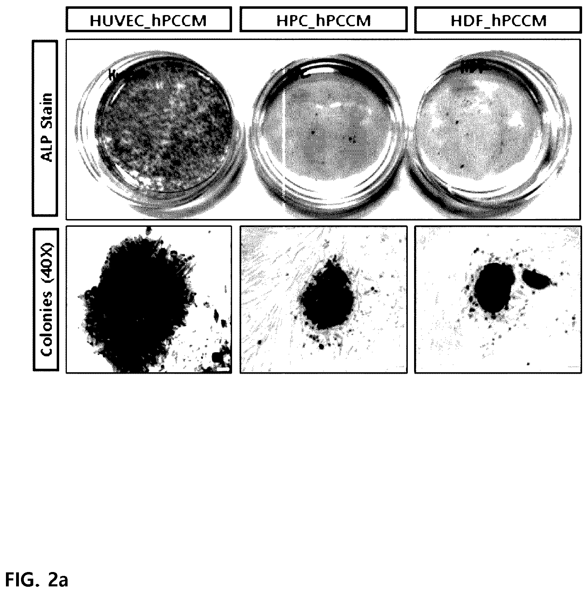

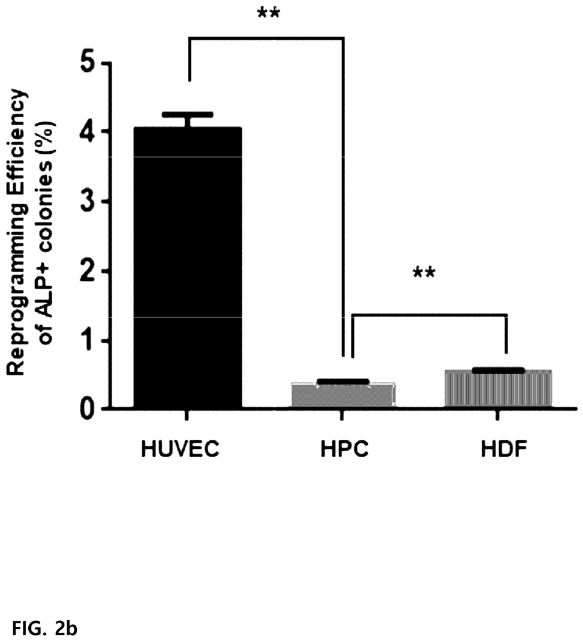

As can be seen in Table 5 and FIGS 2a and 2b, the dedifferentiation efficiency of human endothelial cells was 7 to 10 times as high as that of epithelial cells or placenta cells (**P=0.01).

**TABLE 5**

| Cell | Cell Inoculum (cells/mL) | Colony/well | Efficiency (%) |
|---|---|---|---|
| Human endothelial cell (HUVEC) | 1 X 10⁵ | 4043± 0.21 | 4.043 |
| Placenta cell (HPC) | 1 X 10⁵ | 380 ± 0.03 | 0.381 |
| Epithelial cell (HDF) | 1 X 10⁵ | 553 ± 0.03 | 0.553 |

### EXAMPLE 3: Assay for Expression of CXCR2, mTOR, and β-Catenin in Stem Cells Dedifferentiated from Endothelial Cells, Epithelial Cells, and Placenta Cells

The stem cells dedifferentiated from somatic cells in Example 1 were assayed for expression sites of CXCR2, mTOR, and β-Catenin, using immunofluorescence method. Previous studies reported that inhibition against CXCR2 and mTOR of human pluripotent stem cells decreases the activity of β-Catenin. As for human dedifferentiated stem cells cultured in a placenta-derived cell conditioned medium, the characteristics and growth of the human pluripotent stem cells are controlled through the CXCR2/mTOR/β-Catenin mechanism.

Therefore, it was expected and confirmed that during the formation of induced pluripotent stem cells with a placenta-derived cell conditioned medium, CXCR2 stimulation leads to the activity of dedifferentiated stem cells through the mTOR and β-Catenin mechanism.

In this regard, epithelial cells, endothelial cells, and placenta cells were used as human somatic cells. Sendai virus dedifferentiation factors (OCT4, SOX2, c-Myc, and KLF4, (Life Technologies, CytoTune^{™}-iPS 2.0 Sendai Reprogramming Kit) were purchased and transfected at MOI (multiplicity of infection) of 5 for KOS, at MOI of 5 for c-Myc, and at MOI of 3 for Kif4 into the cells. After transfection, the cells were incubated for 7 days in growth media.

The growth medium for epithelial cells and placenta cells was DMEM (Dulbecco's modified Eagle's medium) supplemented with 10% fetal bovine serum (FBS), 100 U/ml penicillin, and 100 g/ml streptomycin and EBM-2 medium (LONZA, CC-3162) was purchased as the growth medium for endothelial cells.

The transfected somatic cells were transferred to a new culture vessel coated with 0.1% gelatin and supplied with a placenta-derived cell conditioned medium. While colonies were formed during incubation, fluorescence staining was performed every day.

Briefly, the cultured cells were fixed with 4% formalin at room temperature for 10 minutes, permeabilized with 0.1% Triton X-100 for 10 minutes, and then incubated overnight with a 1:1000 dilution of each of primary antibodies to CXCR2 (Abcam #ab14935), Nanog (Santacruz #sc-293121), β-Catenin (Thermo scientific #MA1-2001), and p-mTOR (Cell signaling #5536) at 4°C. On the next day, incubation with a secondary antibody at room temperature for one hour was followed by treatment with 4',6-diamindino-2-phenylindone (DAPI) for 5 minutes in a dark condition before observation by fluorescence microscopy.

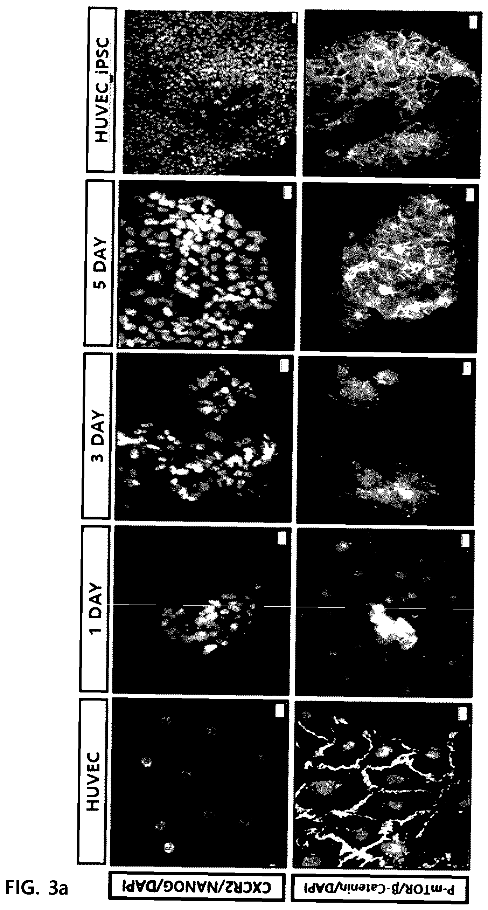

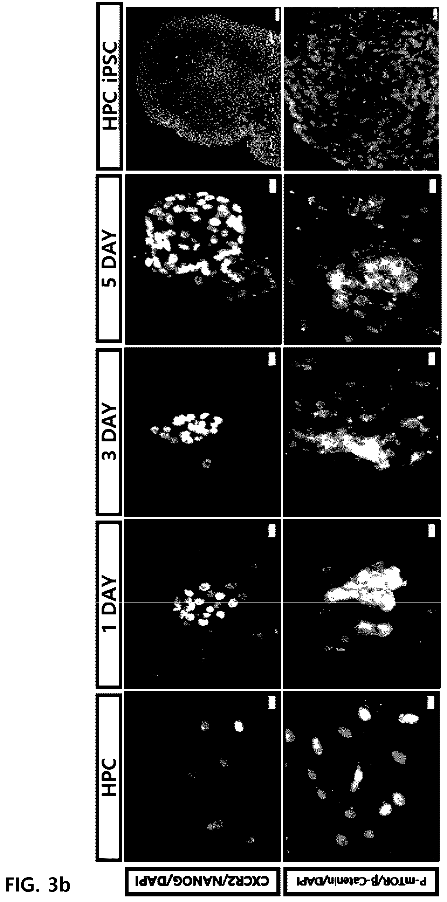

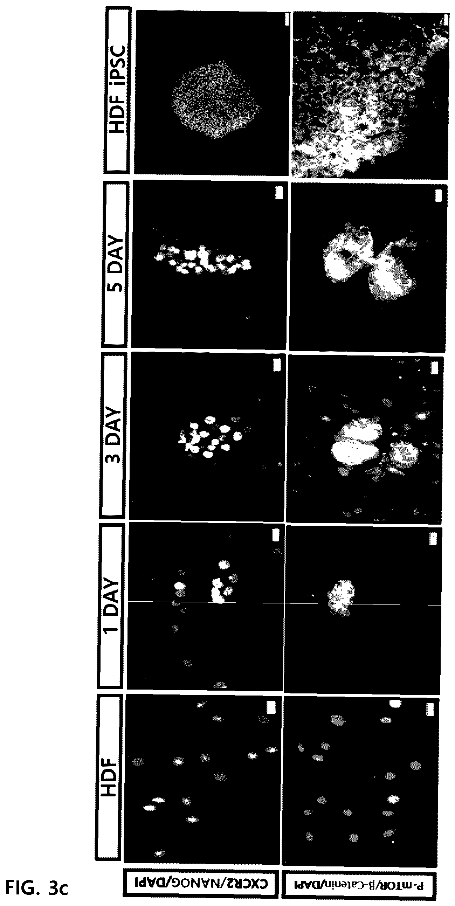

As can be seen in FIGS. 3a to 3c, CXCR2 stimulation to might be considered to allow the somatic cells to have the activity of dedifferentiated stem cells through the mTOR and β-Catenin mechanisms because only the cells which were formed into colonies during the dedifferentiation to stem cells expressed CXCR2, mTOR, and β-Catenin.

### EXAMPLE 4: shRNA-Induced Downregulation of CXCR2 Expression

When the endothelial cells expressing a high level of CXCR2 as in Example 2 were downregulated for CXCR2 expression by shRNA, the efficiency of dedifferentiated stem cells were found to significantly decrease.

For lentiviral infection, endothelial cells were seeded at a density of 1x10⁵ cells/well into 12-well culture plates and incubated. On the next day, the cells were treated with a culture medium containing 6 mg/mL polybrene for 15 minutes and infected with lentivirus (CXCR2 shRNA lentiviral particles, Santacruz #sc-40028-V) having 1x10⁶ TU/mL shRNA inserted thereto. After 24 hours, the medium was replaced with a cell growth medium free of polybrene. From the next day, selection was made of virus-infected cells for 4 to 7 days using 2 µg/mL puromycin. The cells thus harvested were assayed for CXCR2 expression.

Nucleic acid sequences of the CXCR2 shRNA lentiviral particles for use in lentiviral infection are as listed in Table 6, below.

**TABLE 6**

| SEQ ID NO: | Name | Sequence |
|---|---|---|
| 3 | sc-40028-VA | GATCCGTCTACTCATCCAATGTTATTCAAGAGA TAACATTGGATGAGTAGACTTTTT |
| 4 | sc-40028-VB | GATCCCCTCAAGATTCTAGCTATATTCAAGAGA TATAGCTAGAATCTTGAGGTTTTT |
| 5 | sc-40028-VC | GATCCGCCACTAAATTGACACTTATTCAAGAGA TAAGTGTCAATTTAGTGGCTTTTT |

Cell groups which were respectively treated with control lentivirus and CXCR2 lentivirus and not treated with shRNA-inserted lentivirus were measured for CXCR2 expression at protein and RNA levels.

Briefly, for expression measurement at a protein level, cells were washed with chilled PBS and then stirred in a lysis buffer (20 mM KCl, 150 mM NaCl, 1% NP-40, 50 mM NaF, 1 mM DTT, 1 mM EGTA, 1 x protease inhibitor, 10% glycerol, and 50 mM Tris-HCl, pH 7.5) for 15 minutes on ice. The lysate was centrifuged at 14,000 rpm and 4°C for 15 minutes.

The protein concentration in the supernatant was determined using the Bradford assay (Bio-Rad Laboratories, Hercules, CA). Total protein (30 µg) was analyzed by sodium dodecyl sulfate-polyacrylamide gel electrophoresis and transferred onto a nitrocellulose membrane (GE Healthcare Life Sciences, Little Chalfont, UK). The membrane was incubated overnight with a 1:1,000 dilution of a primary antibody to CXCR2 (Abcam #ab65968) at 4°C and then with a 1:2,000 dilution of a HRP-conjugated secondary antibody at room temperature for one hour. Signals were detected using ECL (GE Healthcare Life Sciences).

RNA levels were measured by real-time polymerase chain reaction. For normalization, use was made of GAPDH gene, and P values were used to determine statistical significance (**P =0.05*). Primers for CXCR2 gene expression assay are as listed in Table 7, below.

**TABLE 7**

| SEQ ID NO: | Name | Sequence |
|---|---|---|
| 6 | Forward primer for CXCR2 expression assay | CAATGAATGAATGAATGGCTAAG |
| 7 | Reverse primer for CXCR2 expression assay | AAAGTTTTCAAGGTTCGTCCGTGTT |

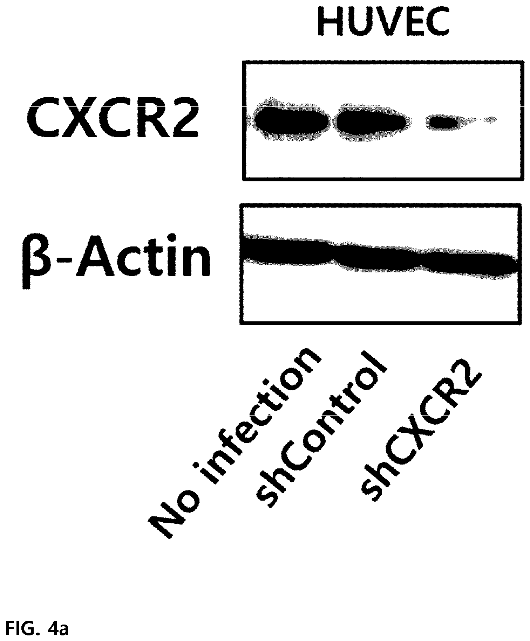

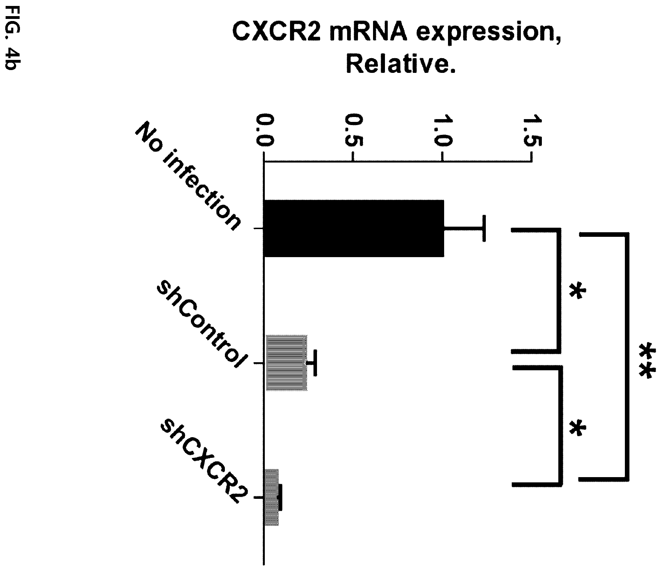

As can be seen in FIGS. 4a and 4b and Table 8, treatment with the shRNA-inserted lentivirus (shCXCR2) downregulated the expression of CXCR2 in the cells derived from endothelial cells.

**TABLE 8**

| | Control (No infection) | shControl | shCXCR2 |
|---|---|---|---|
| Relative mRNA expression level of CXCR2 | 1.00 | 0.26 | 0.08 |

### EXAMPLE 5: Reduction of Dedifferentiation Induction Efficiency by Downregulation of CXCR2 Expression

Alkaline phosphatase (ALP) staining was conducted to examine efficiency of dedifferentiation from somatic cells to induced pluripotent stem cells upon the expression of CXCR2 as in Example 4.

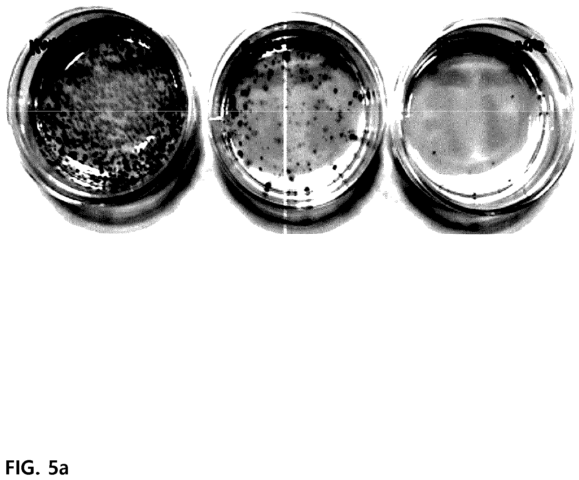

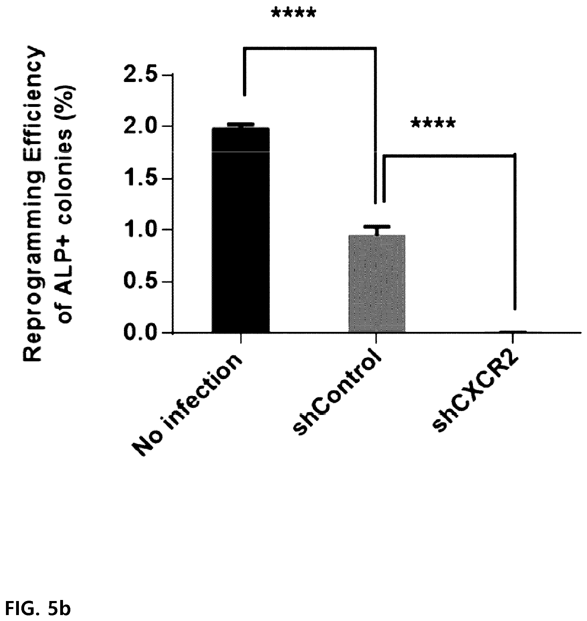

As can be seen in Table 9 and FIGS. 5a and 5b, when endothelial cells were induced to dedifferentiate after the downregulation of CXCR2 expression therein by shCXCR2 lentivirus, efficiency of dedifferentiation to induced pluripotent stem cells was also reduced (****P=0.0001).

**TABLE 9**

| Infection condition | Cell inoculum | Colony/well | Efficiency(%) |
|---|---|---|---|
| Uninfected control | 1 X 10⁵ | 1,974 ± 50 | 1.973 |
| shControl lentivirus | 1 X 10⁵ | 946 ± 89.2 | 0.946 |
| shCXCR2 lentivirus | 1 X 10⁵ | 4 ± 2.70 | 0.004 |

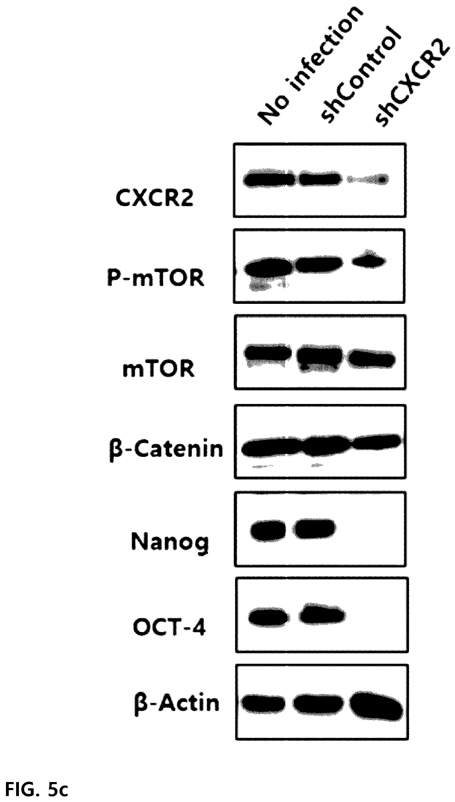

As can be seen in FIG. 5c, when the expression of CXCR2 in endothelial cells was downregulated, the induced pluripotent stem cells dedifferentiated therefrom also decreased in expression levels of induced pluripotent stem cell markers as well as CXCR2, mTOR, and β-Catenin.

### EXAMPLE 6: Increase of Dedifferentiation Induction Efficiency by CXCR2 Overexpression

In epithelial cells, CXCR2 was overexpressed using lentiviral activation particles.

Briefly, epithelial cells were seeded at a density of 1 x 10⁵ cells/well into 12-well plates. After 24 hours, the cells were incubated with 6 µg/ml polybrene for 15 minutes and infected with CXCR2 lentiviral activation particles (IL-8RB Lentiviral Activation Particles (h), SantaCruz, #sc-401404-LAC) at 10 MOI. On the next day, the medium was replaced with a polybrene-free medium and the cells were cultured overnight. Then, only CXCR2 lentiviral activation particle-infected cells were selected and cultured using 5 µg/mL puromycin.

Afterwards, the overexpression was monitored through western blot and real-time polymerase chain reaction. Expression levels of CXCR2 were measured in the same manner as in Example 4.

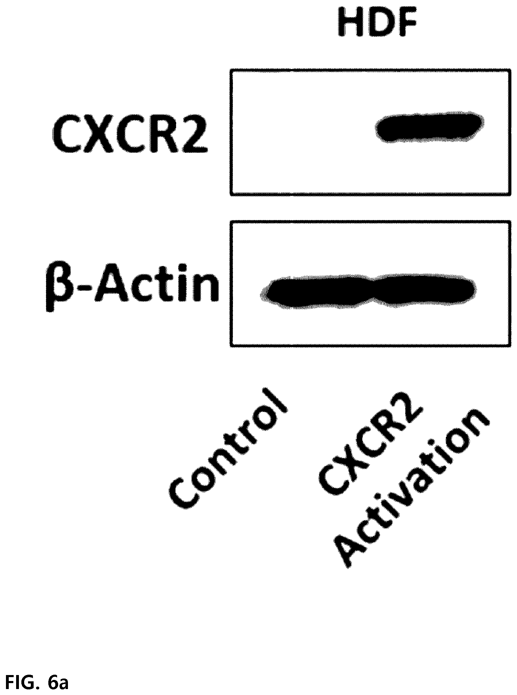

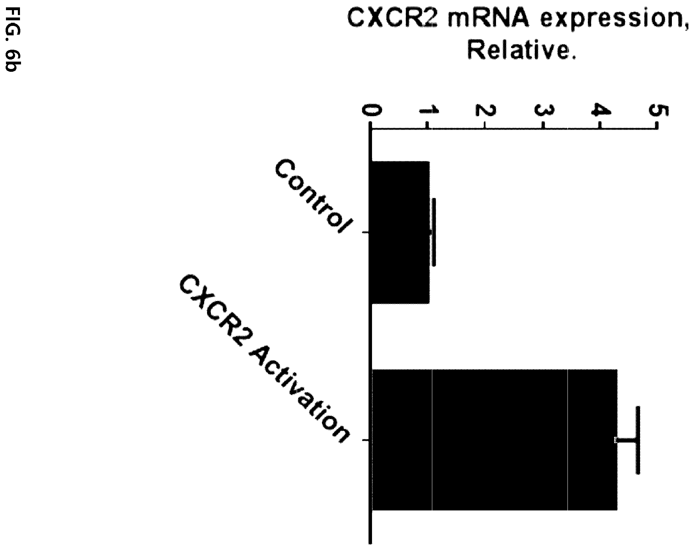

As can be seen in FIGS. 6a and 6b and Table 10, the use of lentiviral activation particles upregulated the expression of CXCR2 in the cells derived from epithelial cells.

**TABLE 10**

| | Control | CXCR2 overexpression (CXCR2 Activation) |
|---|---|---|
| Relative mRNA expression level of CXCR2 | 1.00 | 4.29 |

In order to examine efficiency of dedifferentiation from somatic cells to induced pluripotent stem cells, a CXCR2-overexpressing group and a control group were each cultured in a commercially available pluripotent stem cell culture medium (TeSR) and a placenta-derived cell conditioned medium, separately, followed by conducting alkaline phosphatase staining as in Example 5.

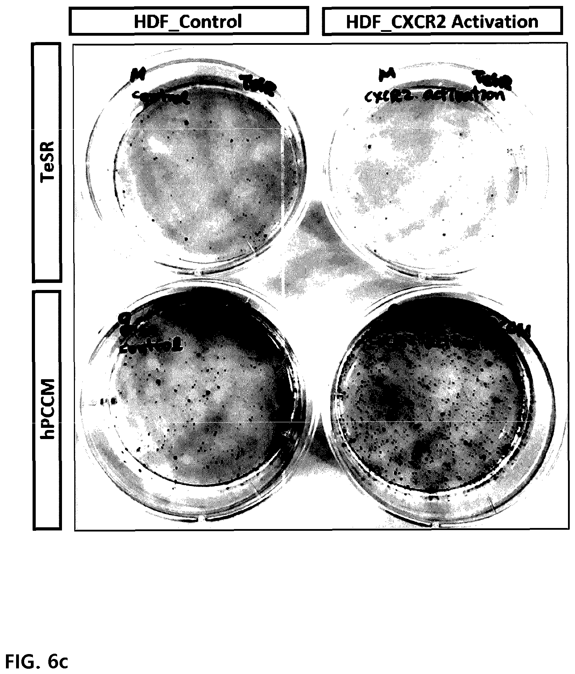

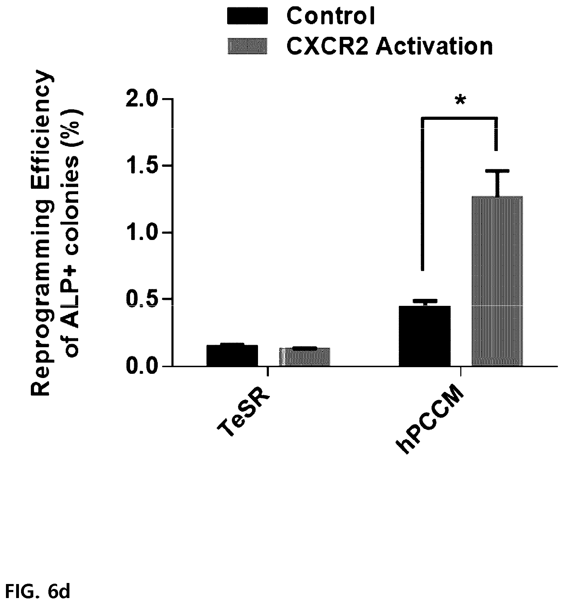

As can be seen in Table 11 and FIGS. 6c and 6d, more induced pluripotent stem cells were dedifferentiated from epithelial cells in which CXCR2 had been overexpressed, compared to the control.

**TABLE 11**

| Infection condition | Cell inoculum | Colony/well | Efficiency (%) |
|---|---|---|---|
| TeSR_control | 1 X 10⁵ | 151 ± 0.09 | 0.15 |
| TeSR_CXCR2 overexpressed | 1 X 10⁵ | 132 ± 0.04 | 0.13 |
| Placenta-derived cell conditioned medium control | 1 X 10⁵ | 445 ± 0.25 | 0.45 |
| Placenta-derived cell conditioned medium _CXCR2 overexpressed | 1 X 10⁵ | 1,267 ± 1.14 | 1.27 |

More dedifferentiated stem cells were established in the placenta-derived cell conditioned medium than in TeSR, with the highest peak of dedifferentiation induction in the group cultured in the placenta-derived cell conditioned medium.

## Claims

1. Use of a composition comprising CXCR2 (CXC chemokine receptor 2) or a ligand thereof for increasing the efficiency of dedifferentiation from a somatic cell to an induced pluripotent stem cell (iPS), wherein the ligand of CXCR2 is at least one selected from the group consisting of GRO-α (growth-regulated oncogene-α), GRO-β (growth-regulated oncogene-β), GRO-γ (growth-regulated oncogene-γ), GCP-2 (granulocyte chemotactic protein-2), NAP-2 (Neutrophil Activating Peptide-2), ENA-78 (Epithelial neutrophil-activating protein-78), and IL-8 (Interleukin-8).

2. The use according to claim 1, wherein the somatic cell is selected from the group consisting of an endothelial cell, an epithelial cell, and a placenta cell.

3. The use according to claim 1, wherein the composition further comprises placenta-derived cell conditioned medium.

4. The use according to claim 3, wherein the placenta-derived cell is a placenta-derived fibroblast-like cell that is isolated from human chorionic plate and cultured.

5. An *in vitro* method for increasing the efficiency of dedifferentiation from a somatic cell to an induced pluripotent stem cell (iPS), the method comprising:
a somatic cell transformation step of increasing the expression of CXCR2; and
a somatic cell culturing step of culturing the transformed somatic cell.

6. The method according to claim 5, wherein the somatic cell transformation step employs a CRISPR/Cas nuclease system or lentiviral activation particles.

7. The method according to claim 5, wherein the somatic cell is at least one selected from the group consisting of an endothelial cell, an epithelial cell, and a placenta cell.

8. The method according to claim 5, wherein the culture is performed in a placenta-derived cell conditioned medium.

9. The method according to claim 8, wherein the placenta-derived cell is a placenta-derived fibroblast-like cell that is isolated from human chorionic plate and cultured.

10. The method according to claim 5, further comprising a stem cell isolation step of isolating a stem cell from a colony formed in the somatic cell culture step.

## Patentansprüche

1. Verwendung einer CXCR2 (CXC-Chemokinrezeptor 2) oder einen Liganden davon umfassenden Zusammensetzung zur Erhöhung der Effizienz der Dedifferenzierung von einer somatischen Zelle zu einer induzierten pluripotenten Stammzelle (iPS), wobei der Ligand von CXCR2 mindestens einer ist, ausgewählt aus der Gruppe bestehend aus GRO-α (wachstumsreguliertes Onkogen-α), GRO-β (wachstumsreguliertes Onkogen-β), GRO-γ (wachstumsreguliertes Onkogen-γ), GCP-2 (Granulozyten-chemotaktisches Protein-2), NAP-2 (Neutrophilen-aktivierendes Peptid-2), ENA-78 (Epitheliales Neutrophilen-aktivierendes Protein-78), und IL-8 (Interleukin-8).

2. Verwendung nach Anspruch 1, wobei die somatische Zelle ausgewählt ist aus der Gruppe bestehend aus einer Endothelzelle, einer Epithelzelle und einer Plazentazelle.

3. Verwendung nach Anspruch 1, wobei die Zusammensetzung ferner ein von der Plazenta stammendes zellkonditioniertes Medium umfasst.

4. Verwendung nach Anspruch 3, wobei die von der Plazenta stammende Zelle eine von der Plazenta stammende fibroblastenähnliche Zelle ist, die aus der menschlichen Chorionplatte isoliert und kultiviert wird.

5. *In-vitro*-Verfahren zur Erhöhung der Effizienz der Dedifferenzierung von einer somatischen Zelle zu einer induzierten pluripotenten Stammzelle (iPS), wobei das Verfahren umfasst:
einen somatischen Zelltransformationsschritt zur Erhöhung der Expression von CXCR2; und
einen somatischen Zellkultivierungsschritt zur Kultivierung der transformierten somatischen Zelle.

6. Verfahren nach Anspruch 5, wobei der Schritt der somatischen Zelltransformation ein CRISPR/Cas-Nuklease-System oder lentivirale Aktivierungspartikel verwendet.

7. Verfahren nach Anspruch 5, wobei die somatische Zelle mindestens eine Zelle ist, ausgewählt ist aus der Gruppe bestehend aus einer Endothelzelle, einer Epithelzelle und einer Plazentazelle.

8. Verfahren nach Anspruch 5, wobei die Kultur in einem von der Plazenta stammenden zellkonditionierten Medium durchgeführt wird.

9. Verfahren nach Anspruch 8, wobei die von der Plazenta stammende Zelle eine von der Plazenta stammende fibroblastenähnliche Zelle ist, die aus der menschlichen Chorionplatte isoliert und kultiviert wird.

10. Verfahren nach Anspruch 5, das ferner einen Schritt der Stammzellenisolierung umfasst, bei dem eine Stammzelle aus einer in dem Schritt der somatischen Zellkultur gebildeten Kolonie isoliert wird.

## Revendications

1. Utilisation d'une composition comprenant CXCR2 (récepteur de chimiokine CXC de type 2) ou un ligand de celui-ci pour augmenter l'efficacité de la dédifférenciation à partir d'une cellule somatique en une cellule souche pluripotente induite (iPS), le ligand de CXCR2 étant au moins un ligand choisi dans le groupe constitué par : GRO-α (oncogène α régulé par la croissance), GRO-β (oncogène β régulé par la croissance), GRO-γ (oncogène γ régulé par la croissance), GCP-2 (protéine chimiotactique de granulocytes-2), NAP-2 (peptide activateur de neutrophiles-2), ENA-78 (protéine épithéliale activatrice de neutrophiles-78) et IL-8 (interleukine-8).

2. Utilisation selon la revendication 1, dans laquelle la cellule somatique est choisie dans le groupe constitué par une cellule endothéliale, une cellule épithéliale et une cellule du placenta.

3. Utilisation selon la revendication 1, dans laquelle la composition comprend en outre un milieu conditionné de cellules dérivées du placenta.

4. Utilisation selon la revendication 3, dans laquelle la cellule dérivée du placenta est une cellule de type fibroblaste dérivée du placenta qui est isolée de la plaque chorionique humaine et cultivée.

5. Procédé *in vitro* pour augmenter l'efficacité de la dédifférenciation à partir d'une cellule somatique en une cellule souche pluripotente induite (iPS), le procédé comprenant :
une étape de transformation de cellule somatique consistant à augmenter l'expression de CXCR2, et
une étape de culture de cellule somatique consistant à cultiver la cellule somatique transformée.

6. Procédé selon la revendication 5, dans lequel l'étape de transformation de cellule somatique utilise un système de nucléase CRISPR/Cas ou des particules d'activation lentivirales.

7. Procédé selon la revendication 5, dans lequel la cellule somatique est au moins une cellule choisie dans le groupe constitué par une cellule endothéliale, une cellule épithéliale et une cellule du placenta.

8. Procédé selon la revendication 5, dans lequel la culture est réalisée dans un milieu conditionné de cellules dérivées du placenta.

9. Procédé selon la revendication 8, dans lequel la cellule dérivée du placenta est une cellule de type fibroblaste dérivée du placenta qui est isolée de la plaque chorionique humaine et cultivée.

10. Procédé selon la revendication 5, comprenant en outre une étape d'isolement de cellule souche consistant à isoler une cellule souche d'une colonie formée lors de l'étape de culture de cellule somatique.
